# EUROPÄISCHE PATENTSCHRIFT

(11) **EP 3 807 639 B1**
(45) Veröffentlichungstag und Bekanntmachung des Hinweises auf die Patenterteilung: **10.09.2025**
(21) Anmeldenummer: 19730773.9
(22) Anmeldetag: 13.06.2019
(51) Int. Cl.: G01N 33/53, G01N 33/543

(54) **VERFAHREN ZUR ENTFERNUNG VON GELÖSTEM SAUERSTOFF BEI EINEM ELEKTROCHEMISCHEN BIOSENSOR DER ALS SENSORENZYM EINE OXIDASE VERWENDET**
METHOD FOR REMOVING DISSOLVED OXYGEN, USED IN AN ELECTROCHEMICAL BIOSENSOR THAT USES AN OXIDASE AS THE SENSOR ENZYME
PROCÉDÉ D'ÉLIMINATION DE L'OXYGÈNE DISSOUS D'UN BIO-CAPTEUR ÉLECTROCHIMIQUE UTILISANT UNE OXYDASE COMME ENZYME DE CAPTEUR

(30) Priorität: 14.06.2018 DE 102018209611
(43) Veröffentlichungstag der Anmeldung: 21.04.2021
(73) Patentinhaber: Ruhr-Universität Bochum, 44801 Bochum (DE)
(72) Erfinder: PLUMERÉ, Nicolas, 44797 Bochum (DE); ZHANG, Huijie Frau, Leeds, LS3 1NP (GB); RUFF, Adrian, 72415 Grosselfingen (DE); VÖPEL, Tobias, 44388 Dortmund (DE)
(74) Vertreter: Kutzenberger Wolff & Partner
(86) Internationale Anmeldenummer: PCT/EP2019/065607
(87) Internationale Veröffentlichungsnummer: WO 2019/238887

(56) Entgegenhaltungen:
- US-A1- 2012 211 372
- US-B2- 9 187 779
- NICOLAS PLUMERÉ ET AL: "Enzyme-Catalyzed O 2 Removal System for Electrochemical Analysis under Ambient Air: Application in an Amperometric Nitrate Biosensor", ANALYTICAL CHEMISTRY, vol. 84, no. 5, 10 February 2012 (2012-02-10), US, pages 2141 - 2146, XP055616634, ISSN: 0003-2700, DOI: 10.1021/ac2020883
- F. LOPEZ ET AL.: "A Polymer Multilayer Based Amperometric Biosensor for the Detection of Lactose in the Presence of High Concentrations of Glucose", ELECTROANALYSIS, 2017, XP002794046
- PABLO A. FIORITO ET AL: "Glucose Amperometric Biosensor Based on the Co-immobilization of Glucose Oxidase (GOx) and Ferrocene in Poly(pyrrole) Generated from Ethanol / Water Mixtures", JOURNAL OF THE BRAZILIAN CHEMICAL SOCIETY, vol. 12, no. 6, 1 January 2001 (2001-01-01), pages 729 - 733, XP055617973, DOI: 10.1590/S0103-50532001000600007
- EUN-HYUNG YOO ET AL: "Glucose Biosensors: An Overview of Use in Clinical Practice", SENSORS, vol. 10, no. 5, 4 May 2010 (2010-05-04), pages 4558 - 4576, XP055424345, DOI: 10.3390/s100504558

## Beschreibung

### Stand der Technik

Die vorliegende Erfindung geht aus von einem Verfahren zum Bestimmen einer Konzentration eines Analyten in einer Probe. Solche Verfahren sind aus dem Stand der Technik grundsätzlich bekannt und werden viele Millionen Mal am Tag durchgeführt. Als Beispiele seien hier Kontrollen im Bereich der Lebensmittelproduktion und Blutglukosemessungen erwähnt. Allein von letzteren werden in Deutschland 24 Millionen Stück pro Tag an Patienten mit einer Diabetes Mellitus Erkrankung durchgeführt.

Zum Messen einer Konzentration eines Analyten, beispielsweise einer Glukosekonzentration oder einer Laktosekonzentration, werden üblicherweise elektrochemische Biosensoren eingesetzt, welche unter Zuhilfenahme von enzymatischen Reaktionen geeignet sind, die zu messenden Konzentrationen zu ermitteln. Eine große Herausforderung bei diesen Verfahren ist, dass in der Probe gelöster Sauerstoff die Messergebnisse beeinflusst. Bei elektrochemischen Biosensoren führt die Anwesenheit von gelöstem Sauerstoff zu einem Kurzschluss des Elektronentransferprozesses vom Analyten zur Elektrode. Dies führt bei geringen Konzentrationen des Analyten zu erheblichen Ungenauigkeiten.

Zwar ist die Verwendung von Tests zur Ermittlung der Glukosekonzentration im Blut von Diabetespatienten dringend notwendig und kann Leben retten, jedoch stehen die verwendeten Verfahren immer wieder wegen mangelhafter Genauigkeit kontrovers in der Diskussion. Die auf dem Markt am weitesten verbreiteten Biosensoren für Glukose basieren auf den Enzymen Glukosedehydrogenase oder Glukoseoxidase. Beide Enzyme haben unterschiedliche Nachteile die die Genauigkeit der Messung beeinflussen können. So liefern einige der Glukosedehydrogenase basierten Systeme falsche Messwerte in Anwesenheit bestimmter Medikamente und reagieren unspezifisch mit anderen Zuckern wie Maltose, Galaktose und Xylose. Dadurch kommt es zu falsch erhöhten Messwerten die zu einer Nichterkennung von Hypoglykämien mit Todesfolge führen können.

Testsysteme auf Glukoseoxidase Basis sind weniger anfällig für Medikamente, unterschiedliche pH-Werte und die Temperatur des Blutes des Patienten und auf Grund ihrer Spezifizität für Glukose nicht anfällig für andere Zucker. Darüber hinaus ist Glukoseoxidase günstig herzustellen. Jedoch weisen Glukoseoxidase Testsysteme eine Empfindlichkeit gegenüber erhöhten oder erniedrigten Sauerstoffkonzentrationen auf. So führt eine erhöhte Sauerstoffkonzentration zu niedrigeren Messwerten, wohingegen eine erniedrigte Sauerstoffkonzentration in überhöhten Messwerten resultiert. Ferner kommt es in Anwesenheit von Sauerstoff zur Bildung von Wasserstoffperoxid, welches die verwendete Glukoseoxidase deaktivieren kann.

Aus dem Stand der Technik sind Methoden zur Sauerstoffentfernung bekannt, bei denen Glukoseoxidase, Galaktoseoxidase oder Pyranoseoxidase mit Katalase kombiniert wurden (US9187779B2). Durch die beiden Enzyme wird die Entfernung von Sauerstoff ermöglicht. Dabei katalysiert die Oxidase die Oxidation eines Zuckers (Glukose, Galaktose) zu einem Halbacetal (Glukoseoxidase), Keton (Pyranoseoxidase) oder Aldehyd (Galaktoseoxidase) und Wasserstoffperoxid. Das dabei entstehende Wasserstoffperoxid wird durch die zugesetzte Katalase zu Sauerstoff und Wasser abgebaut. Diese Kombination erlaubt die effektive Entfernung von Sauerstoff in Anwesenheit der entsprechenden Zucker. Für die Entwicklung eines Biosensors zur Detektion von Glukose kann dieses System aber nicht genutzt werden, da die verwendeten Oxidoreduktasen (außer Galaktoseoxidase) spezifisch für Glukose sind. Ein Abbau von Glukose würde das Messergebnis eines Glukose-Biosensors jedoch verfälschen. Ein weiterer Nachteil der beschriebenen Methode ist, dass die verwendeten Oxidoreduktasen (Glukoseoxidase, Galaktoseoxidase und Pyranoseoxidase) die Elektronen auch an einen Mediator des Messprozesses übertragen können, was zur Verfälschung der Messergebnisse führen würde.

Zum Stand der Technik gehört weiterhin ein Verfahren bei dem eine Oxidase zur Entfernung von Sauerstoff in einem Oxidase basiertem elektrochemischen Sensorsystem eingesetzt wurde. Dabei wird eine räumliche der Trennung beider Prozesse (O₂ Entfernung und Detektion des Analyten) vorgenommen ("A Polymer Multilayer Based Amperometric Biosensor for the Detection of Lactose in the Presence of High Concentrations of Glucose" von F. Lopez et al., "Electroanalysis" 2017). Die räumliche Trennung wird dabei durch die Verwendung von mehreren Lagen realisiert. Eine untere Lage ist elektrisch mit einer Elektrode verbunden und kann Elektronen übertragen. In dieser Lage befindet sich ein Enzym welches spezifisch für den Analyten ist. Auf die untere Lage wird eine obere Schicht aufgebracht in welche Enzyme zur Entfernung von Sauerstoff oder anderen Molekülen wie zum Beispiel Glukose und Wasserstoffperoxid eingebettet sind. Dieser Aufbau hat jedoch den Nachteil, dass das Sensorenzym in der unteren Lage lokalisiert ist. Durch diesen Aufbau können Diffusionsprozesse die Konzentration des Analyten in der unteren Lage limitieren und die Auftragung der oberen Schicht kann die Aktivität der Enzyme in der unteren Lage beeinträchtigen.

Weiterhin sind aus dem Stand der Technik bereits Methoden zur Sauerstoffentfernung mit Alkoholoxidase bekannt. Dabei wird die Alkoholoxidase in Bakterien oder Pilzen hergestellt. Diese Zellen werden dann als Ganzes, als Zelllysat oder als Zellextrakt zur Entfernung von Sauerstoff verwendet. Bei dieser Verwendung sind die Zellen oder Extrakte in eine sauerstoffpermeable Membran verpackt. Die Verwendung von Zellen oder Zellextrakten ist aber für die Entwicklung eines Biosensors nicht praktikabel, da sich diese schwer in einen miniaturisierten Sensor integrieren lassen und eher für die Entfernung von Sauerstoff in Verpackungen geeignet sind.

Aus der US 2012/0211372 A1 sind Systeme und Verfahren zur enzymatischen Entfernung von Sauerstoff aus wässrigen Lösungen bekannt. Diese Systeme und Verfahren eignen sich jedoch nicht zur Anwendung in Biosensoren, in denen der Gehalt eines Analyten mittels Oxidasen bestimmt wird.

### Offenbarung der Erfindung

Es ist daher eine Aufgabe der vorliegenden Erfindung ein Verfahren bereitzustellen, welches gelösten Sauerstoff aus einer wässrigen Lösung entfernt und die oben geschilderten Nachteile des Standes der Technik nicht aufweist.

Diese Aufgabe wird durch ein Verfahren gemäß Anspruch 1 realisiert.

Das erfindungsgemäße Verfahren nutzt eine Oxidase zur Sauerstoffentfernung.

Der gelöste Sauerstoff wird über eine gekoppelte Enzymreaktion mit einer Oxidase und einer Katalase entfernt. **In** einem ersten Schritt wird eine erste Reaktion von einer Oxidase katalysiert, wobei in der ersten Reaktion Sauerstoff mit einem Substrat der Oxidase, z.B. einem kurzkettigen Alkohol oder Formaldehyd, zu einem Aldehyd oder einer Säure und Wasserstoffperoxid reagiert, in einem zweiten Schritt in einer zweiten Reaktion beispielsweise von einer Katalase katalysiert das Wasserstoffperoxid zu Wasser und ½ Sauerstoff disproportioniert wird. Der entstandene Sauerstoff wird durch zyklisches Durchlaufen des ersten und des zweiten Schrittes endgültig zu Wasser umgewandelt. Die verwendete Oxidase ist hoch spezifisch für Sauerstoff als Elektronen Akzeptor und es kommt zu keiner Übertragung von Elektronen auf Mediatoren oder andere nicht spezifische Moleküle (Azevedo, Biosensors&Bioelectronics, 2005). Durch diese besondere Eigenschaft der Oxidase ist sichergestellt, dass die Entfernung von Sauerstoff keinen Einfluss auf die Messergebnisse von enzymbasierten Biosensoren, genauer oxidasebasierten Biosensoren, hat. Durch das hier beschriebene Verfahren wird der gelöste Sauerstoff schnell und vollständig entfernt. Durch die gekoppelte Enzymreaktion ist es möglich, gelösten Sauerstoff auch aus kleinen Probenvolumina effektiv zu entfernen ohne, dass es in mehr als vernachlässigbarem Maße durch Diffusionsprozesse von Sauerstoff zu direkten Reaktionen an der Elektrode eines elektrochemischen Biosensors kommen kann.

Das erfindungsgemäße Verfahren eignet sich in vorteilhafter Weise zum Entfernen von gelöstem Sauerstoff aus wässrigen Lösungen, insbesondere zum Entfernen von gelöstem Sauerstoff aus wässrigen Lösungen zum Verbessern der Qualität von Ergebnissen von elektrochemischen Analysen, die Oxidasen spezifisch für den gewünschten Analyten als Sensorenzym verwenden, zum Beispiel bei der Bestimmung von Glukosekonzentrationen.

Alle Komponenten, die für die Sauerstoffentfernung und die Bestimmung eines Analyten notwendig sind, können als Feststoff auf der Elektrode einer elektrochemischen Messzelle abgeschieden werden. Die verwendeten Enzyme können durch Tropfguss (engl. Drop-Casting) aufgebracht und anschließend getrocknet werden. Das Substrat für die Oxidase zur Sauerstoffentfernung - beispielsweise Methanol, Ethanol oder Propanol - kann zum Beispiel durch Vermischen mit Maltodextrin zu einem wasserlöslichen Feststoff verarbeitet werden. Substrat im Sinne der vorliegenden Erfindung umfasst insbesondere das Reduktionsmittel. Methanol kann außerdem durch Zugabe von Calciumacetat zu einem wasserlöslichen Gel verarbeitet werden. Paraformaldehyd (CAS-Nr: 30525-89-4), ein weiteres Substrat für die Oxidase zur Sauerstoffentfernung, ist ein Feststoff der ebenfalls in einer elektrochemischen Messzelle verwendet werden kann. Das erfindungsgemäße Verfahren hat den Vorteil, dass durch Zugabe einer wässrigen Probe, die den Analyten enthält, alle notwendigen Komponenten in Lösung gebracht werden, wodurch keine arbeitsintensive Probenvorbereitung notwendig ist. Außerdem verbessert die Verwendung von Feststoffen in vorteilhafter Weise die Effizienz der Konzentrationsbestimmung eines Analyten mit Hilfe einer Analyt-spezifischen Oxidase, da die Feststoffe keinen Einfluss auf die Oxidationsreaktion des Sensorenzyms und die elektrochemischen Prozesse der Messzelle haben.

Denkbar ist es weiterhin, das erfindungsgemäße Verfahren bei anderen chemischen Arbeitsgängen zu nutzen, bei denen gelöster Sauerstoff ein Störfaktor ist, beispielsweise bei der Konzentrationsanalyse von Phosphaten oder Laktose mit Hilfe von oxidase-basierten Biosensoren. Die Qualität der Ergebnisse von elektrochemischen Analysen mit sauerstoffempfindlichen Sensorenzymen, insbesondere wenn es sich bei den verwendeten Enzymen um Oxidasen handelt wird durch das erfindungsgemäße Verfahren verbessert.

Vorteilhafte Ausgestaltungen und Weiterbildungen der Erfindung sind den Unteransprüchen, sowie der Beschreibung unter Bezugnahme auf die Zeichnungen entnehmbar.

Gemäß einer bevorzugten Ausführungsform der vorliegenden Erfindung ist vorgesehen, dass als Reduktionsmittel ein kurzkettiger Alkohol, vorzugsweise Methanol oder Ethanol oder Propanol oder Butanol oder Pentanol oder Formaldehyd oder Paraformaldehyd verwendet wird. Denkbar ist, den Alkohol in einer Konzentration von 5 bis 100 mmol/l, bevorzugt von 45 mmol/I bis 55 mmol/I zu verwenden. Als weiteres mögliches Substrat für die Oxidase zur Sauerstoffentfernung kann Paraformaldehyd verwendet werden. Paraformaldehyd ist ein Polmyer von Formaldehyd und liegt als Feststoff vor.

Gemäß einer weiteren bevorzugten Ausführungsform der vorliegenden Erfindung ist vorgesehen, dass als Oxidase zur Sauerstoffentfernung eine Alkoholoxidase, welche aus Pichia pastoris, Candida boidinii, Hansenula sp. oder anderen Organismen isoliert ist, verwendet wird. Die genannten Oxidasen verbessern in vorteilhafter Weise die Wirkung des erfindungsgemäßen Verfahrens. Diese Oxidasen besitzen eine hohe spezifische Aktivität für kurzkettige Alkohole und Formaldehyd und sind zudem günstig und kommerziell verfügbar. Die vorgesehene Oxidase wird so ausgewählt, dass die enzymatische Aktivität für die Oxidation eines Analyten deutlich geringer ist als für die Oxidation des Reduktionsmittels. Die enzymatische Aktivität für die Oxidation des Analyten beträgt dabei weniger als 5% der enzymatischen Aktivität der Oxidation des Reduktionsmittels.

Gemäß einer weiteren bevorzugten Ausführungsform der vorliegenden Erfindung ist vorgesehen, dass die Disproportionierung mittels Katalysator, vorzugsweise mittels Katalase, besonders bevorzugt mittels Katalase, welche aus Rinderleber isoliert ist erfolgt. Aus Rinderleber isolierte Katalase verbessert in vorteilhafter Weise die Effizienz des erfindungsgemäßen Verfahrens. Ferner ist diese Katalase kommerziell verfügbar. Denkbar ist aber auch, dass Aspergillus niger, Corynebacterium glutamicum oder Micrococcus lysodeikticus als Katalase verwendet werden.

Ein weiterer Gegenstand der vorliegenden Erfindung ist ein Messsystem zur elektrochemischen Bestimmung eines Analyten in einer wässrigen Lösung in einer elektrochemischen Messzelle, wobei die Messzelle Elektroden und die chemischen Reaktionspartner zur Durchführung eines erfindungsgemäßen Verfahrens aufweist. Das Messsystem ermöglicht es, elektrochemische Messungen durchzuführen, wobei zur Verbesserung der Genauigkeit der Messung gelöster Sauerstoff mit dem Verfahren nach einem der Ansprüche 1 bis 4 entfernt wird. Dabei wird gelöster Sauerstoff entfernt, ohne dass der Prozess der Sauerstoffentfernung die elektrochemische Bestimmung mit Hilfe einer Oxidase beeinflussen würde.

Gemäß einer bevorzugten Ausführungsform der vorliegenden Erfindung ist vorgesehen, dass wobei die chemischen Reaktionspartner in die Messzelle integriert sind.

Gemäß einer bevorzugten Ausführungsform der vorliegenden Erfindung ist vorgesehen, dass die Elektroden Siebdruckelektroden sind. Diese sind günstig und flexibel in der Herstellung und Verwendung. Denkbar ist auch, als Elektroden Glaskohlenstoff-Elektroden einzusetzen. Die Elektroden können wenige Millimeter groß, bevorzugt bis zu 3 mm groß, sein.

Gemäß einer bevorzugten Ausführungsform der vorliegenden Erfindung ist vorgesehen, dass das Volumen der elektrochemischen Messzelle ≤ 150 µl, vorzugsweise ≤ 100 µl ist. In einer ebenfalls bevorzugten Ausführungsform der vorliegenden Erfindung kann das Volumen im Bereich von ≥ 0,5µl bis ≤ 100 µl, vorzugsweise ≥ 0,5µl bis ≤ 50 µl, besonders bevorzugt ≥ 0,5µl bis ≤ 10 µl liegen.

Gemäß einer weiteren bevorzugten Ausführungsform der vorliegenden Erfindung ist vorgesehen, dass das Messsystem 1,1-Ferrocendimethanol als Elektronenmediator aufweist. Denkbar ist auch, dass als Sensorenzym ein Enzym der Klasse Oxidase (EC-Nummer: 1.1.3) und einem zum Sensorenzym kompatiblen Elektronenmediator aufweist. Denkbar ist weiterhin, dass der Elektronenmediator ein ähnliches Redox-Potenzial aufweist wie das Sensorenzym. Denkbar ist weiterhin, dass weitere Ferrocen-Derivate als Elektronenmediator verwendet werden. Denkbar ist aber auch, andere Klassen von Elektronenmediatoren wie zum Beispiel Ferricyanide, leitende organische Salze, Redox-Hydrogele mit einem komplexierten Metall (z.B. Eisen oder Osmium oder Ruthenium), Phenothiazine, Phenoxazine oder Viologene zu verwenden. Durch die Verwendung einer Oxidase aus P. Pastoris können die oben genannten Elektronenmediatoren verwendet werden, weil dieses Enzym nicht mit diesen reagiert, sondern spezifisch für Sauerstoff als Elektronenakzeptor ist.

Gemäß einer weiteren bevorzugten Ausführungsform der vorliegenden Erfindung ist vorgesehen, dass das Messsystem ein Redox-Polymer mit einem Osmium-Komplex als Elektronenmediator aufweist.

Gemäß einer weiteren bevorzugten Ausführungsform der vorliegenden Erfindung ist vorgesehen, dass der Analyt Glukose, Laktose und/oder Phosphat ist, wobei das Sensorenzym zur Oxidation des Analyten Glukoseoxidase, Laktoseoxidase oder eine Kombination aus Xanthin Oxidase und Purin-Nukleosid-Phosphorylase aufweist. Damit ist es möglich, die Konzentration an Analyt, z.B. die Glukosekonzentration, Laktosekonzentration oder Phosphatkonzentration einer wässrigen Lösung zu messen. Die jeweilige Oxidase, z.B. die Glukoseoxidase, wird so ausgewählt, dass die enzymatische Aktivität für die Oxidation des Reduktionsmittels deutlich geringer ist als für die Oxidation des Analyten. Die enzymatische Aktivität für die Oxidation des Reduktionsmittels beträgt dabei weniger 5% der enzymatischen Aktivität der Oxidation des Analyten.

Gemäß einer bevorzugten Ausführungsform der Erfindung verursacht die Übertragung von Elektronen auf den Elektronenmediator, durch die Oxidase zur Sauerstoffentfernung, einen gemessenen Strom der ≤5% des Stromes beträgt der bei der Oxidation des Analyten gemessen wird. Um dies zu gewährleisten ist ein spezifisches Konzentrationsverhältnis zwischen der Oxidase zur Sauerstoffentfernung und dem Sensorenzym zu bestimmen.

Denkbar wäre aber auch, dass das Messsystem Laktoseoxidase zur Bestimmung von Laktose oder eine Kombination von Xanthinoxidase und Purin-Nukleosid-Phosphorylase für die Bestimmung von Phosphat aufweist. Weiterhin ist es aber auch denkbar, dass eine Ausführungsform der vorliegenden Erfindung eine Oxidase aufweist, die für einen noch nicht in den vorherigen Ausführungsformen genannten Analyten spezifisch ist.

Ein weiterer Gegenstand der vorliegenden Erfindung ist ein Verfahren zur elektrochemischen Bestimmung einer Konzentration eines Analyten in einer wässrigen Lösung mit einem Messsystem gemäß einem der Ansprüche 5 bis 11, dadurch gekennzeichnet, dass gelöster Sauerstoff in der wässrigen Lösung mit einem Verfahren gemäß einem der Ansprüche 1 bis 4 entfernt wird. Das Verfahren ermöglicht es, die Konzentration eines Analyten in einer wässrigen Lösung zu bestimmen, ohne dass vorhandener gelöster Sauerstoff zu unerwünschten Abweichungen der Messergebnisse führt

Gemäß einer bevorzugten Ausführungsform der vorliegenden Erfindung ist vorgesehen, dass der Analyt unter Katalyse durch eine Oxidase in einer Oxidationsreaktion oxidiert wird, wobei Elektronen aus der Oxidationsreaktion von einem Elektronenmediator zu einer Elektrode transportiert werden, wobei der durch den Transport der Elektronen durch den Elektronenmediator entstehende Strom gemessen wird. Dies ermöglicht es, die Konzentration des Analyten anhand eines Stromes zwischen den Elektroden des verwendeten Messsystems zu bestimmen.

Gemäß einer weiteren bevorzugten Ausführungsform der vorliegenden Erfindung ist vorgesehen, dass der Analyt Glukose ist, wobei als Oxidase Glukoseoxidase verwendet wird. Hiermit wird ein Verfahren zur Verfügung gestellt, eine Glukosekonzentration in einer wässrigen Lösung zu bestimmen, ohne dass gebundener Sauerstoff oder die Produktion von Wasserstoffperoxid zu verfälschten Ergebnissen führen würde. Denkbar wäre aber auch, dass für das Verfahren Laktoseoxidase zur Bestimmung von Laktose oder eine Kombination von Xanthinoxidase und Purin-Nukleosid-Phosphorylase für die Bestimmung von Phosphat verwendet wird. Weiterhin ist aber auch denkbar, dass eine Oxidase verwendet wird, die für einen noch nicht in den vorherigen Ausführungsformen genannten Analyten spezifisch ist.

Gemäß einer weiteren bevorzugten Ausführungsform der vorliegenden Erfindung ist vorgesehen, dass der Analyt Laktose oder Phosphat ist, wobei als Oxidase Lactatoxidase oder eine Kombination aus Xanthin Oxidase und Purin-Nukleosid-Phosphorylase verwendet wird.

Gemäß einer weiteren bevorzugten Ausführungsform der vorliegenden Erfindung ist vorgesehen, dass als Elektronenmediator 1,1-Ferrocendimethanol verwendet wird. Gemäß einer weiteren bevorzugten Ausführungsform der vorliegenden Erfindung ist vorgesehen, dass als Elektronenmediator ein Redox-Polymer mit einem Osmium-Komplex verwendet wird. Es hat sich gezeigt, dass 1,1-Ferrocenedimethanol oder ein Redox-Hydrogel mit komplexiertem Osmium als Elektronenmediator die Qualität der Messergebnisse des Verfahrens erhöht. Denkbar ist auch, dass andere Klassen von Elektronenmediatoren, wie zum Beispiel Ferricyanide, leitende organische Salze, Metallkomplexe (z.B. Eisen oder Osmium oder Ruthenium Komplexe), Phenothiazine, Phenoxazine oder Viologene, verwendet werden.

Weitere Einzelheiten, Merkmale und Vorteile der Erfindung ergeben sich aus den Zeichnungen, sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den wesentlichen Erfindungsgedanken nicht einschränken.

### Kurze Beschreibung der Zeichnung

- Figur 1: zeigt das Grundprinzip des Verfahrens zur Entfernung von gelöstem Sauerstoff gemäß einer beispielhaften Ausführungsform der vorliegenden Erfindung.
- Figur 2: zeigt das Funktionsprinzip des elektrochemischen Biosensors zur Bestimmung der Konzentration des Analyten der wässrigen Probe, bei der gelöster Sauerstoff entfernt wird gemäß einer beispielhaften Ausführungsform der vorliegenden Erfindung.
- Figur 3: zeigt die elektrochemische Charakterisierung verschiedener kurzkettigen Alkohole als Reduktionsmittel für die Oxidase zur Sauerstoffentfernung.
- Figur 4: zeigt die elektrochemische Charakterisierung von Maltodextrin-Methanol-Feststoff als Reduktionsmittel für die Oxidase zur Sauerstoffentfernung.
- Figur 5: zeigt die elektrochemische Charakterisierung von Calcium Acetat-Methanol-Feststoff als Reduktionsmittel für die Oxidase zur Sauerstoffentfernung.
- Figur 6: zeigt die elektrochemische Charakterisierung von Paraformaldehyd als Reduktionsmittel für die Oxidase zur Sauerstoffentfernung.
- Figur 7: zeigt die elektrochemische Charakterisierung des Elektronenmediators 1,1 Ferrocendimethanol zusammen mit der Oxidase zur Sauerstoffentfernung.
- Figur 8: zeigt die elektrochemische Charakterisierung der Oxidase zur Sauerstoffentfernung mit dem Elektronenmediator 1,1 Ferrocendimethanol in Lösung. Die Oxidase kann keine Elektronen auf diesen Mediator übertragen.
- Figur 9: zeigt die elektrochemische Charakterisierung der Oxidase zur Sauerstoffentfernung mit Glucose in der Lösung. Die Oxidase zur Sauerstoffentfernung kann Glucose nicht oxidieren.
- Figur 10: zeigt die Bestimmung einer Konzentrationsreihe von Glucose unter verschiedenen Bedingungen in einem Probenvolumen von 1000 µl.
- Figur 11: zeigt die den Einfluss verschiedener Konzentrationsverhältnisse der Oxidase zur Sauerstoffentfernung und des Sensorenzyms.
- Figur 12: zeigt die Bestimmung einer Konzentrationsreihe von Glucose unter verschiedenen Bedingungen in einem Probenvolumen von 100 µl.

### Beispielhafte Ausführungsform der Erfindung

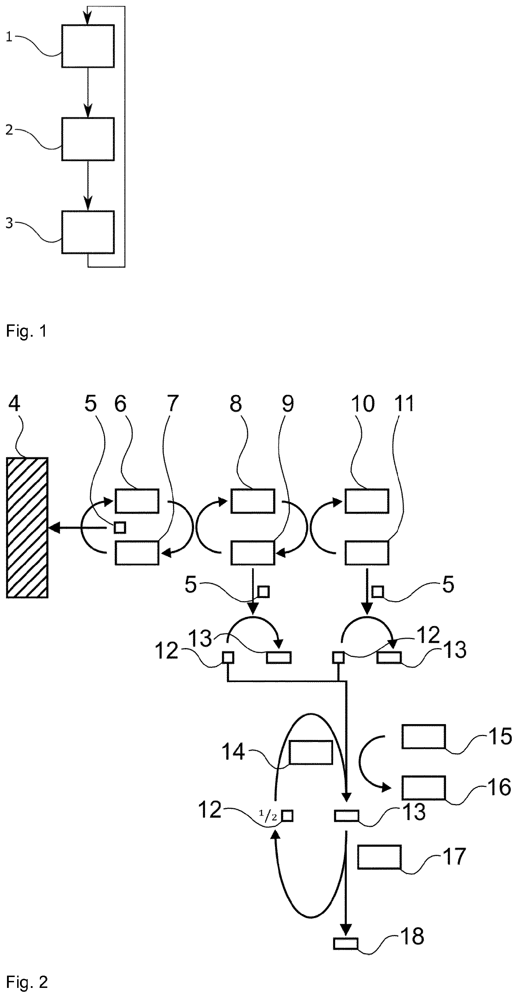

In **Figur 1** ist das Grundprinzip des Verfahrens zur Entfernung von gelöstem Sauerstoff 12 gemäß einer beispielhaften Ausführungsform der vorliegenden Erfindung schematisch als Ablaufdiagramm dargestellt. Im ersten Schritt 1 reagiert ein Reduktionsmittel 15, hier Methanol, mit Sauerstoff 12 zu einem Aldehyd 16 und Wasserstoffperoxid 13. Als Katalysator dieser Reaktion dient eine Oxidase zur Sauerstoffentfernung 14, hier Alkoholoxidase. Im zweiten Schritt 2 wird das Wasserstoffperoxid 13 zu Wasser 18 reduziert und die Katalase 17 oxidiert. Ferner werden die oxidierte Katalase 17 und weiterhin Wasserstoffperoxid 13 zu Wasser 18 und Sauerstoff 12 reduziert. Die Summengleichung des zweiten Schrittes 2 lautet 2 H₂O₂ → O₂ + 2 H₂O. Nach Durchlaufen des ersten Schrittes 1 und des zweiten Schrittes 2 hat sich 1 O₂ auf ½ O₂ verringert. Durch vielfaches Durchlaufen des ersten Schrittes 1 und des zweiten Schrittes 2 wird so der gelöste Sauerstoff 12 entfernt.

In **Figur 2** ist das Grundprinzip des Verfahrens zur Entfernung von gelöstem Sauerstoff 12 gemäß einer beispielhaften der vorliegenden Erfindung schematisch als Ablaufdiagramm dargestellt.

Die Elektrode 4 nimmt im ersten Schritt 1 des Verfahrens ein Elektron 5 auf und wandelt den reduzierten Mediator 7 in die oxidierte Form um. Das oxidierte Sensorenzym 8 (z.B. Glukoseoxidase) oxidiert den Analyten 11 (z.B. Glukose) und wird dabei zum reduzierten Sensorenzym 9 reduziert. Ist der Analyt 11 Glukose, so entsteht Glucono-Lakton als oxidierter Analyt 10.

Als Nebenreaktion kann das reduzierte Sensorenzym 9 durch gelösten Sauerstoff 12 unter Abgabe eines Elektrons 5 oxidiert werden. Dabei wird Wasserstoffperoxid 13 freigesetzt, welches der an der Elektrode 4 eine Interferenz herbeiführt.

Durch das erfindungsgemäße Verfahren kann der gelöste Sauerstoff 12 vollständig entfernt werden. Dabei reagiert das Reduktionsmittel 15, hier ein Alkohol, mit dem Sauerstoff 12 zu dem Aldehyd 16 und Wasserstoffperoxid 13. Das Reduktionsmittel 15 kann in verschiedenen alternativen Ausführungsformen Ethanol, Propanol, Butanol, Pentanol und bevorzugt Methanol sein. Als Katalysator dieser Reaktion dient eine Oxidase zur Sauerstoffentfernung 14, hier Alkoholoxidase. Die verwendete Oxidase zur Sauerstoffentfernung 14 ist sehr selektiv für Sauerstoff 12 und nutzt keine künstlichen Elektronenmediatoren zur Übertragung von Elektronen 5. Diese Eigenschaft ermöglicht es, Sauerstoff 12 bei oxidasebasierten Biosensoren zu entfernen, ohne mit diesem zu interferieren. Im zweiten Schritt 2 wird das Wasserstoffperoxid 13 zu Wasser 18 reduziert und die Katalase 17 oxidiert. Weiterhin werden die oxidierte Katalase 17 und das Wasserstoffperoxid 13 zu Wasser 18 und ½ Sauerstoff 12 reduziert. Nach Durchlaufen des ersten Schrittes 1 und des zweiten Schrittes 2 hat sich 1 Sauerstoff 12 auf ½ Sauerstoff 12 verringert. Durch vielfaches zyklisches Durchlaufen des ersten Schrittes 1 und des zweiten Schrittes 2 wird so der gelöste Sauerstoff 12 vollständig entfernt.

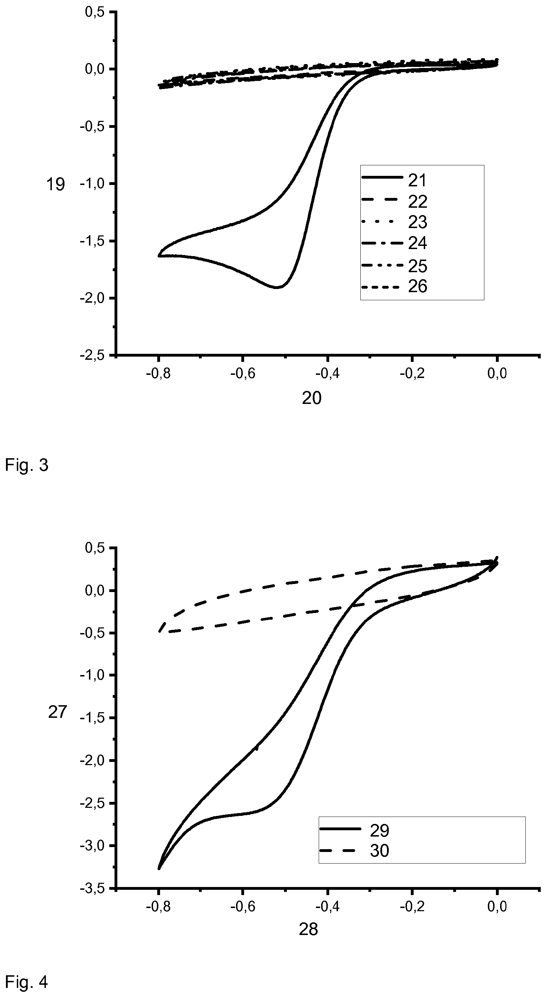

Die Oxidase zur Sauerstoffentfernung 14 oxidiert ein Substrat und entfernt dabei gelösten Sauerstoff 12. In **Figur 3** ist die Entfernung von Sauerstoff 12 mit Hilfe von verschiedenen Substraten gezeigt. Gemäß einer beispielhaften Ausführungsform der vorliegenden Erfindung kann Methanol oder Ethanol oder Propanol oder Butanol oder Pentanol als Substrat mit einer Konzentration von 50 mmol/I verwendet werden. Gemäß einer bevorzugten Ausführungsform ist vorgesehen den Alkohol in fester Form zu Verfügung zu stellen. **In** **Figur 4** ist die Entfernung von Sauerstoff 12 mit dem Reduktionsmittel 15 Methanol gebunden in Maltodextrin gezeigt. Dazu wird das Reduktionsmittel 15 solange zu Maltodextrin gegeben, bis das Pulver keine Flüssigkeit mehr binden kann. Der Überschuss an Methanol wird durch Evaporation entfernt. Das für das Verfahren gebundene Reduktionsmittel-Maltodextrin beinhaltet vorzugsweise circa 5 Gewichtsprozent Methanol. Der Feststoff wird dem Reaktionsgemisch vor Beginn der Reaktion zugegeben (1 mg auf 100 µl). Der Sauerstoff 12 wird dabei genauso effektiv entfernt wie durch die Zugabe eines flüssigen Substrates (vgl. Figur 3).

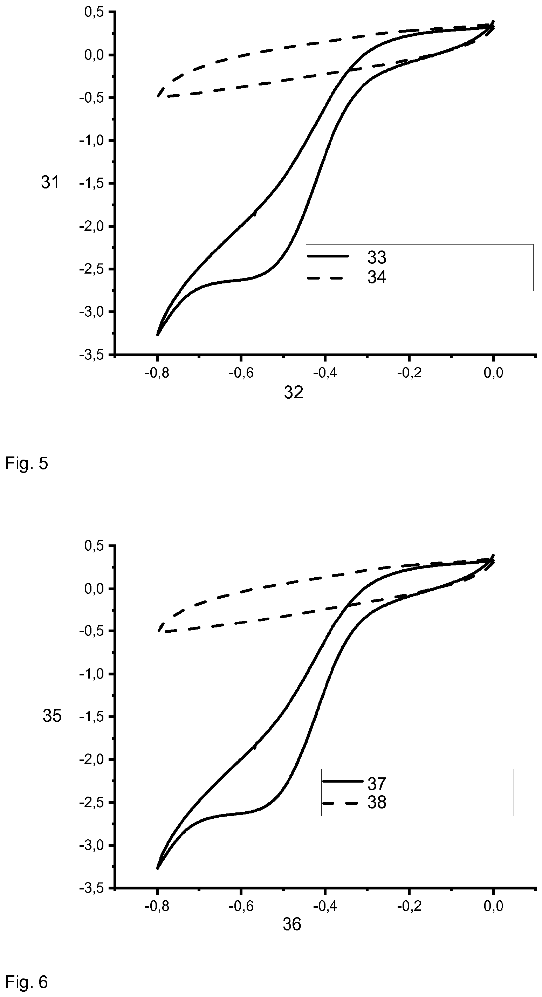

In **Figur 5** ist die Entfernung von Sauerstoff 12 mit dem Reduktionsmittel 15 Methanol gebunden in Calciumacetat gezeigt. Dazu wird eine gesättigte Lösung von Calciumacetat hergestellt (3 g Ca(CH₃COO)₂ zu 10 g Wasser). Anschließend wird unter Rühren 40 ml Methanol hinzugegeben. Anschließend wird 2 mg dieses Feststoffes zu 100 µl Reaktionsgemisch gegeben. Der Sauerstoff 12 wird dabei genauso effektiv entfernt wie durch die Zugabe von flüssigem Substrat.

In einer weiteren beispielhaften Ausführungsform der vorliegenden Erfindung wird Paraformaldehyd, ein Polymer von Formaldehyd, als festes Reduktionsmittel 15 zur Sauerstoffentfernung verwendet. Dazu wird 1 mg Paraformaldehyd zu 100 µl Reaktionsgemisch gegeben. **Figur** 6 zeigt die elektrochemische Charakterisierung der Sauerstoffentfernung. Der Sauerstoff 12 wird genauso effektiv entfernt wie durch die Zugabe von flüssigem Substrat.

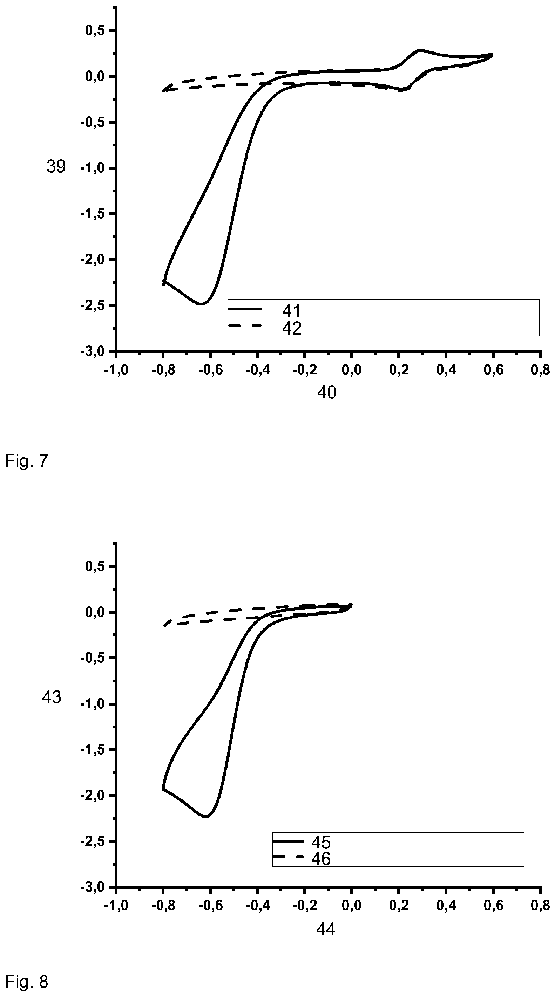

Die Oxidase zur Sauerstoffentfernung 14 ist nicht in der Lage, Elektronen 5 auf einen Elektronenmediator zu übertragen. Das in **Figur 7** gezeigte Diagramm enthält experimentelle Daten eines Experiments bei dem der Einfluss auf die Sauerstoffentfernung der Oxidase untersucht wurde. Dieses Experiment hat zeigt, dass die Oxidase zur Sauerstoffentfernung 14 keine Elektronen 5 auf den Elektronenmediator 1,1 Ferrocendimethanol überträgt, jedoch weiterhin den Sauerstoff 12 aus der Lösung entfernen kann.

Die Oxidase zur Sauerstoffentfernung 14 ist nicht in der Lage, den Analyten 11 zu oxidieren. **Figur 8** zeigt ein Diagramm mit Daten aus einem Experiment bei dem Glukose als Substrat eines erfindungsgemäßen Biosensors hinzugegeben wird. Die Oxidase zur Sauerstoffentfernung 14 ist in der Lage den Sauerstoff 12 aus der Lösung vollständig zu entfernen. Die zugegebene Glukose zeigt keinen Einfluss auf den Hintergrundstrom eines Kontrollexperiments (vgl. Figur 3).

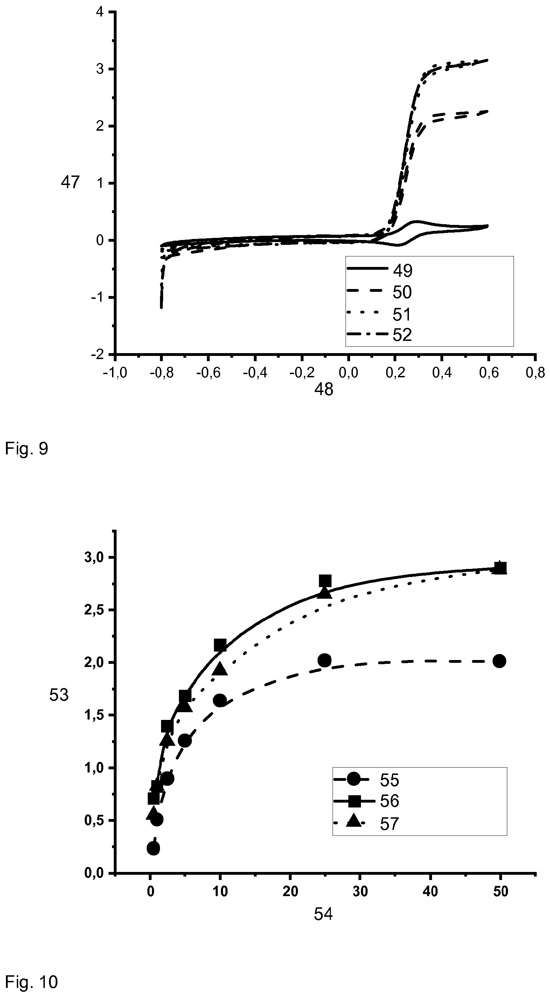

Um die Einsatzfähigkeit der Oxidase zur Sauerstoffentfernung 14 in einem Biosensorsystem zu demonstrieren wird ein Biosensor für Glukose unter verschiedenen Bedingungen getestet. Die Daten dieser Testreihe sind in **Figur 9** in Diagrammform dargestellt. Der Strom für die Oxidation des Analyten 11 Glucose durch das Sensorenzym wurde an einer Luftatmosphäre, einer Argonatmosphäre und an einer Raumluftatmosphäre in Kombination mit der Oxidase zur Sauerstoffentfernung 14 bei einer Glukosekonzentration von 50 mmol/I untersucht. Anhand des Vergleiches dieser Messungen ist zu erkennen, dass der gemessene Strom unter Argonatmosphäre identisch mit dem Strom ist der gemessen wurde, wenn die Oxidase zur Sauerstoffentfernung bei einer Raumluftatmosphäre eingesetzt wurde. Im Vergleich dazu ist der gemessene Strom niedriger, wenn die Oxidase zur Sauerstoffentfernung 14 nicht im System vorhanden ist. In **Figur 10** ist eine Konzentrationsreihe eines Glukose-Biosensors dargestellt. Die Anwesenheit von Sauerstoff 12 in der Lösung führt zu bis zu 30% geringeren gemessenen Strömen da das Sensorenzym Elektronen 5 auf den in der Lösung vorhandenen Sauerstoff 12 überträgt.

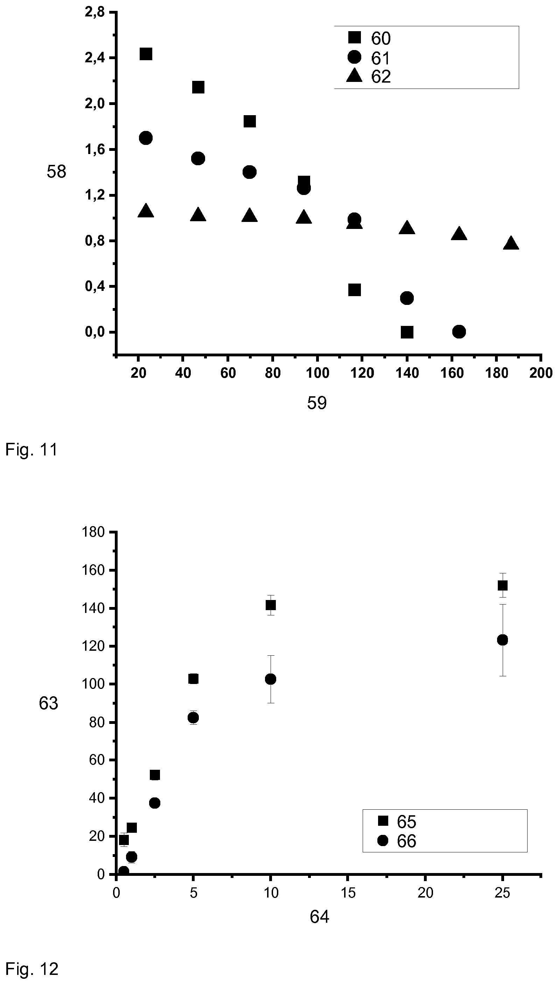

Ein bevorzugtes Verhältnis zwischen dem Sensorenzym und der Oxidase zur Sauerstoffentfernung 14 ist 1:10. In **Figur 11** ist der experimentelle Nachweis dieser Optimierung dargestellt. Dies kann durch einen konstanten Strom über eine Messzeit von 200 Minuten gezeigt werden. Der konstante Strom zeigt an, dass Sauerstoff 12, der aus der Luft in die Lösung diffundiert, sofort und vollständig entfernt werden kann. **Figur 12** zeigt die Detektion einer Konzentrationsreihe von Glukose in einem Reaktionsvolumen von 100 µl eines sauerstoff-unempfindlichen Biosensors. Die Interferenz von Sauerstoff 12 in der Lösung führt dazu, dass der gemessene Strom im Durchschnitt 25% und maximal 90% (~10 mmol/I Glukosekonzentration) geringer ist.

### Bezugszeichenliste

**Fig. 1**
   - 1: Erster Schritt
   - 2: Zweiter Schritt
   - 3: Dritter Schritt
**Fig. 2**
   - 4: Elektrode
   - 5: Elektron
   - 6: Oxidierter Mediator
   - 7: Reduzierter Mediator
   - 8: Oxidiertes Sensorenzym
   - 9: Reduziertes Sensorenzym
   - 10: Oxidierter Analyt
   - 11: Analyt
   - 12: Sauerstoff
   - 13: Wasserstoffperoxid
   - 14: Oxidase zur Sauerstoffentfernung
   - 15: Reduktionsmittel
   - 16: Aldehyd
   - 17: Katalase
   - 18: Wasser
**Fig. 3**
   - 19: Y-Achse: Strom in µA
   - 20: X-Achse: Potential in V (E vs Ag/AgCl 3M KCI)
   - 21: Kontrolle in Phosphatpuffer (100 mM, pH 7,0-7,5)
   - 22: 1 ml Phosphatpuffer + 50 mmol/I Methanol
   - 23: 1 ml Phosphatpuffer + 50 mmol/I Ethanol
   - 24: 1 ml Phosphatpuffer + 50 mmol/I Propanol
   - 25: 1 ml Phosphatpuffer + 50 mmol/I Butanol
   - 26: 1 ml Phosphatpuffer + 50 mmol/I Pentanol
**Fig. 4**
   - 27: Y-Achse: Strom in µA
   - 28: X-Achse: Potential in V (E vs Ag/AgCl 3M KCI)
   - 29: Kontrolle in 1 ml Phosphatpuffer (100 mM, pH 7,0-7,5)
   - 30: 1 ml Phosphatpuffer + Maltodextrin-Methanol Feststoff
**Fig. 5**
   - 31: Y-Achse: Strom in µA
   - 32: X-Achse: Potential in V (E vs Ag/AgCl 3M KCI)
   - 33: Kontrolle in 1 ml Phosphatpuffer (100 mM, pH 7,0-7,5)
   - 34: 1 ml Phosphatpuffer + Calciumacetat-Methanol Feststoff
**Fig. 6**
   - 35: Y-Achse: Strom in µA
   - 36: X-Achse: Potential in V (E vs Ag/AgCl 3M KCI)
   - 37: Kontrolle in 1 ml Phosphatpuffer (100 mM, pH 7,0-7,5)
   - 38: 1 ml Phosphatpuffer + Paraformaldehyd (0,1 mg/ml)
**Fig. 7**
   - 39: Y-Achse: Strom in µA
   - 40: X-Achse: Potential in V (E vs Ag/AgCl 3M KCI)
   - 41: 1 ml Phosphatpuffer (100 mM, pH 7,0-7,5), 10U AOX, 2000U CAT, 100 µmol/l 1,1-Ferrocendimethanol
   - 42: 1 ml Phosphatpuffer (100 mM, pH 7,0-7,5), 10U AOX, 2000U CAT, 100 µmol/l 1,1-Ferrocendimethanol, 50 mmol/I Ethanol
**Fig. 8**
   - 43: Y-Achse: Strom in µA
   - 44: X-Achse: Potential in V (E vs Ag/AgCl 3M KCI)
   - 45: 1 ml Phosphatpuffer (100 mM, pH 7,0-7,5), 10U AOX, 2000U CAT, 50 mmol/I Glukose
   - 46: 1 ml Phosphatpuffer (100 mM, pH 7,0-7,5), 10U AOX, 2000U CAT, 50 mmol/I Glukose, 50 mmol/I Ethanol
**Fig. 9**
   - 47: Y-Achse: Strom in µA
   - 48: X-Achse: Potential in V (E vs Ag/AgCl 3M KCI)
   - 49: Kontrolle ohne Glukose: 1 ml Phosphatpuffer (100 mM, pH 7,0-7,5), 10U AOX, 2000U CAT, 15U GOX, 100 µmol/l 1,1-Ferrocendimethanol, 50 mmol/I Ethanol
   - 50: Glukosebestimmung Luftatmosphäre: 1 ml Phosphatpuffer (100 mM, pH 7,0-7,5), 15U GOX, 100 µmol/l 1,1-Ferrocendimethanol, 50 mmol/I Glukose
   - 51: Glukosebestimmung mit Sauerstoffentfernung Luftatmosphäre: 1 ml Phosphatpuffer (100 mM, pH 7,0-7,5), 10U AOX, 2000U CAT, 15U GOX, 100 µmol/l 1,1-Ferrocendimethanol, 50 mmol/I Ethanol, 50 mmol/I Glukose
   - 52: Glukosebestimmung Argonatmosphäre: 1 ml Phosphatpuffer (100 mM, pH 7,0-7,5), 15U GOX, 100 µmol/l 1,1-Ferrocendimethanol, 50 mmol/I Glukose
**Fig. 10**
   - 53: Y-Achse: Strom in µA
   - 54: X-Achse: Glukosekonzentration im mmol/I
   - 55: Kontrolle Luftatmospähre: 1 ml Phosphatpuffer (100 mM, pH 7,0-7,5), 15U GOX, 100 µmol/l 1,1 Ferrocendimethanol
   - 56: Glukosebestimmung mit Sauerstoffentfernung Luftatmosphäre: 1 ml Phosphatpuffer (100 mM, pH 7,0-7,5), 10U AOX, 2000U CAT, 15U GOX, 100 µmol/l 1,1-Ferrocendimethanol, 50 mmol/I Ethanol
   - 57: Glukosebestimmung Argonatmosphäre: 1 ml Phosphatpuffer (100 mM, pH 7,0-7,5), 15U GOX, 100 µmol/l 1,1-Ferrocendimethanol
**Fig. 11**
   - 58: y-Achse: Strom in µA
   - 59: X-Achse: Zeit in Minuten
   - 60: Glukosebestimmung mit Sauerstoffentfernung Luftatmosphäre: 1 ml Phosphatpuffer (100 mM, pH 7,0-7,5), 10U AOX, 2000U CAT, 15U GOX, 100 µmol/l 1,1-Ferrocendimethanol, 100 mmol/I Ethanol
   - 61: Glukosebestimmung mit Sauerstoffentfernung Luftatmosphäre: 1 ml Phosphatpuffer (100 mM, pH 7,0-7,5), 20U AOX, 2000U CAT, 10U GOX, 100 µmol/l 1,1-Ferrocendimethanol, 100 mmol/I Ethanol
   - 62: Glukosebestimmung mit Sauerstoffentfernung Luftatmosphäre: 1 ml Phosphatpuffer (100 mM, pH 7,0-7,5), 50U AOX, 2000U CAT, 5U GOX, 100 µmol/l 1,1-Ferrocendimethanol, 100 mmol/I Ethanol
**Fig. 12**
   - 63: y-Achse: Strom in µA
   - 64: X-Achse: Glukosekonzentration in mmol/l
   - 65: Glukosebestimmung mit Sauerstoffentfernung Luftatmosphäre: 1 ml Phosphatpuffer (100 mM, pH 7,0-7,5), 10U AOX, 2000U CAT, 15U GOX, 100 µmol/l 1,1-Ferrocendimethanol, 100 mmol/I Ethanol
   - 66: Glukosebestimmung mit Sauerstoffentfernung Argonatmosphäre: 1 ml Phosphatpuffer (100 mM, pH 7,0-7,5), 2000U CAT, 10U GOX, 100 µmol/l 1,1-Ferrocendimethanol

## Patentansprüche

1. Verfahren zur Entfernung von gelöstem Sauerstoff (12) bei einem elektrochemischen Biosensor der eine erste Oxidase zur Sauerstoffentfernung (14) verwendet und eine zweite Oxidase als Sensorenzym (8/9) verwendet, wobei
i) in einem ersten Schritt (1) eine erste Reaktion von der ersten Oxidase zur Sauerstoffentfernung (14) katalysiert wird, wobei in der ersten Reaktion Sauerstoff mit einem Reduktionsmittel (15) zu Wasserstoffperoxid (13) reagiert, ohne dass Elektronen (5) auf einen anderen Akzeptor als Sauerstoff (12) übertragen werden, wobei keine anderen Komponenten des elektrochemischen Biosensors oxidiert werden,
ii) in einem zweiten Schritt (2) in einer zweiten Reaktion das Wasserstoffperoxid (13) zu Wasser (18) disproportioniert wird,
iii) in einem dritten Schritt (3) in einer dritten Reaktion die zweite Oxidase, die als Sensorenzym (8/9) dient, die Oxidation des Analyten (11) katalysiert, ohne dass durch den dritten Schritt (3) andere Komponenten einer wässrigen Lösung des elektrochemischen Biosensors oxidiert werden, wobei die Oxidase die als Sensorenzym (8/9) dient, spezifisch für den Analyten (11) ist, wobei die durch die Oxidation des Analyten (11) freigesetzten Elektronen (5) ausschließlich an einen Elektronenmediator übertragen werden.

2. Verfahren nach Anspruch 1, wobei als Reduktionsmittel (15) ein kurzkettiger Alkohol, vorzugsweise Methanol oder Ethanol oder Propanol oder Butanol oder Pentanol, oder Formaldehyd oder Paraformaldehyd verwendet wird.

3. Verfahren nach einem der vorhergehenden Ansprüche, wobei als erste Oxidase zur Sauerstoffentfernung (14) eine Oxidase, welche aus *Pichia pastoris* oder *Candida boidinii* oder *Hansenula sp.* isoliert ist, verwendet wird.

4. Verfahren nach einem der vorhergehenden Ansprüche, wobei die Disproportionierung von Wasserstoffperoxid (13) zu Wasser (18) mittels Katalysator, vorzugsweise mittels Katalase (17), erfolgt; und als Katalase (17) bevorzugt eine Katalase verwendet wird, welche aus Rinderleber isoliert ist.

5. Messsystem zur elektrochemischen Bestimmung eines Analyten (11) in einer wässrigen Lösung in einer elektrochemischen Messzelle, wobei die Messzelle Elektroden (4) aufweist, wobei die Messzelle die chemischen Reaktionspartner zur Durchführung eines Verfahrens gemäß einem der vorhergehenden Ansprüche aufweist.

6. Messsystem nach Anspruch 5, wobei die chemischen Reaktionspartner in die Messzelle integriert sind.

7. Messsystem nach einem der Ansprüche 5 oder 6, wobei die Elektroden (4) Siebdruckelektroden sind.

8. Messsystem nach einem der Ansprüche 5 bis 7, wobei das Volumen der Messzelle kleiner als ≤ 150 µl, vorzugsweise ≤ 100 µl ist.

9. Messsystem nach einem der Ansprüche 5 bis 8, wobei das Messsystem 1,1-Ferrocendimethanol als Elektronenmediator aufweist.

10. Messsystem nach einem der Ansprüche 5 bis 9, wobei das Messsystem ein Redox-Polymer mit einem Osmium-Komplex als Elektronenmediator aufweist.

11. Messsystem nach einem der Ansprüche 5 bis 10, wobei der Analyt (11) Glukose, Laktose und/oder Phosphat ist, wobei das Sensorenzym zur Oxidation des Analyten Glukoseoxidase, Laktoseoxidase oder eine Kombination aus Xanthin Oxidase und Purin-Nukleosid-Phosphorylase aufweist.

12. Verfahren zur elektrochemischen Bestimmung einer Konzentration eines Analyten (11) in einer wässrigen Lösung mit einem Messsystem gemäß einem der Ansprüche 5 bis 11, **dadurch gekennzeichnet, dass** gelöster Sauerstoff (12) in der wässrigen Lösung mit einem Verfahren gemäß einem der Ansprüche 1 bis 4 entfernt wird.

13. Verfahren gemäß Anspruch 12, wobei der Analyt (11) unter Katalyse durch die Oxidase in einer Oxidationsreaktion oxidiert wird, wobei Elektronen (5) aus der Oxidationsreaktion von dem Elektronenmediator zu einer Elektrode (4) transportiert werden, wobei der durch den Transport der Elektronen (5) durch den Elektronenmediator entstehende Strom gemessen wird.

14. Verfahren gemäß Anspruch 12 oder 13, wobei der Analyt (11)
- Glukose ist, wobei als Oxidase Glukoseoxidase verwendet wird; oder
- Laktose oder Phosphat ist, wobei als Oxidase Lactatoxidase oder eine Kombination aus Xanthin Oxidase und Purin-Nukleosid-Phosphorylase verwendet wird.

15. Verfahren nach einem der Ansprüche 12 bis 14, wobei als Elektronenmediator
- 1,1-Ferrocenedimethanol verwendet wird; oder
- ein Redox-Polymer mit einem Osmium-Komplex verwendet wird.

## Claims

1. Method for removing dissolved oxygen (12) in an electrochemical biosensor which uses a first oxidase for oxygen removal (14) and a second oxidase as a sensor enzyme (8/9), wherein
i) in a first step (1), a first reaction is catalyzed by the first oxidase for oxygen removal (14), wherein in the first reaction, oxygen reacts with a reducing agent (15) to form hydrogen peroxide (13) without electrons (5) being transferred to an acceptor other than oxygen (12), wherein no other components of the electrochemical biosensor are oxidized,
ii) in a second step (2), in a second reaction, the hydrogen peroxide (13) is disproportionated to water (18),
iii) in a third step (3), in a third reaction, the second oxidase, which serves as a sensor enzyme (8/9), catalyzes the oxidation of the analyte (11) without the third step (3) oxidizing other components of an aqueous solution of the electrochemical biosensor, wherein the oxidase that serves as a sensor enzyme (8/9) is specific for the analyte (11), wherein the electrons (5) released by the oxidation of the analyte (11) are transferred exclusively to an electron mediator.

2. Method according to claim 1, wherein a short-chain alcohol, preferably methanol or ethanol or propanol or butanol or pentanol, or formaldehyde or paraformaldehyde is used as the reducing agent (15).

3. Method according to one of the preceding claims, wherein an oxidase isolated from *Pichia pastoris* or *Candida boidinii* or *Hansenula sp.* is used as the first oxidase for oxygen removal (14).

4. Method according to one of the preceding claims, wherein the disproportionation of hydrogen peroxide (13) to water (18) is carried out by means of a catalyst, preferably by means of catalase (17); and a catalase isolated from bovine liver is preferably used as catalase (17).

5. Measuring system for the electrochemical determination of an analyte (11) in an aqueous solution in an electrochemical measuring cell, wherein the measuring cell has electrodes (4), wherein the measuring cell has the chemical reaction partners for carrying out a method according to one of the preceding claims.

6. Measuring system according to claim 5, wherein the chemical reaction partners are integrated into the measuring cell.

7. Measuring system according to one of claims 5 or 6, wherein the electrodes (4) are screen-printed electrodes.

8. Measuring system according to one of claims 5 to 7, wherein the volume of the measuring cell is less than ≤ 150 µl, preferably ≤ 100 µl.

9. Measuring system according to one of claims 5 to 8, wherein the measuring system comprises 1,1-ferrocendimethanol as an electron mediator.

10. Measuring system according to one of claims 5 to 9, wherein the measuring system comprises a redox polymer with an osmium complex as an electron mediator.

11. Measuring system according to one of claims 5 to 10, wherein the analyte (11) is glucose, lactose and/or phosphate, wherein the sensor enzyme for oxidizing the analyte comprises glucose oxidase, lactose oxidase or a combination of xanthine oxidase and purine nucleoside phosphorylase.

12. Method for the electrochemical determination of a concentration of an analyte (11) in an aqueous solution using a measuring system according to one of claims 5 to 11, **characterized in that** dissolved oxygen (12) in the aqueous solution is removed using a method according to one of claims 1 to 4.

13. Method according to claim 12, wherein the analyte (11) is oxidized in an oxidation reaction catalyzed by the oxidase, wherein electrons (5) from the oxidation reaction are transported from the electron mediator to an electrode (4), wherein the current generated by the transport of the electrons (5) through the electron mediator is measured.

14. Method according to claim 12 or 13, wherein the analyte (11)
- is glucose, wherein glucose oxidase is used as the oxidase; or
- lactose or phosphate, wherein lactate oxidase or a combination of xanthine oxidase and purine nucleoside phosphorylase is used as the oxidase.

15. Method according to any of claims 12 to 14, wherein the electron mediator is
- 1,1-ferrocenedimethanol is used as the electron mediator; or
- a redox polymer with an osmium complex is used.

## Revendications

1. Procédé d'élimination d'oxygène dissous (12) dans un biocapteur électrochimique utilisant une première oxydase pour éliminer l'oxygène (14) et une seconde oxydase comme enzyme de détection (8/9), dans lequel
i) dans une première étape (1), une première réaction est catalysée par la première oxydase pour éliminer l'oxygène (14), dans lequel dans la première réaction, l'oxygène réagit avec un agent réducteur (15) pour former du peroxyde d'hydrogène (13) sans que les électrons (5) soient transférés à un accepteur autre que l'oxygène (12), dans lequel aucun autre composant du biocapteur électrochimique n'est oxydé,
ii) dans une deuxième étape (2), dans une deuxième réaction, le peroxyde d'hydrogène (13) est disproportionné en eau (18),
iii) dans une troisième étape (3), dans une troisième réaction, la deuxième oxydase servant d'enzyme de détection (8/9) catalyse l'oxydation de l'analyte (11) sans que d'autres composants d'une solution aqueuse du biocapteur électrochimique soient oxydés par la troisième étape (3), dans lequel l'oxydase sert d'enzyme de détection (8/9) est spécifique de l'analyte (11), dans lequel les électrons (5) libérés par l'oxydation de l'analyte (11) est transmis exclusivement à un médiateur d'électrons.

2. Procédé selon la revendication 1, dans lequel on utilise comme agent réducteur (15) un alcool à chaîne courte, de préférence le méthanol ou l'éthanol ou le propanol ou le butanol ou le pentanol, ou le formaldéhyde ou le paraformaldéhyde.

3. Procédé selon l'une quelconque des revendications précédentes, dans lequel on utilise comme première oxydase pour éliminer l'oxygène (14) une oxydase isolée de *Pichia pastoris* ou de *Candida boidinii* ou de *Hansenula sp.*

4. Procédé selon l'une quelconque des revendications précédentes, dans lequel la disproportionation du peroxyde d'hydrogène (13) par rapport à l'eau (18) est effectuée au moyen d'un catalyseur, de préférence au moyen d'une catalase (17) ; et la catalase (17) utilisée est de préférence une catalase isolée à partir de foie de bovin.

5. Système de mesure pour la détermination électrochimique d'un analyte (11) en solution aqueuse dans une cellule de mesure électrochimique, dans lequel la cellule de mesure comprend des électrodes (4), dans lequel la cellule de mesure comprend les réactifs chimiques pour la mise en œuvre d'un procédé selon l'une quelconque des revendications précédentes.

6. Système de mesure selon la revendication 5, dans lequel les réactifs chimiques sont intégrés dans la cellule de mesure.

7. Système de mesure selon l'une des revendications 5 ou 6, dans lequel les électrodes (4) sont des électrodes sérigraphiques.

8. Système de mesure selon l'une des revendications 5 à 7, dans lequel le volume de la cellule de mesure est inférieur à ≤ 150 µl, de préférence ≤ 100 µl.

9. Système de mesure selon l'une quelconque des revendications 5 à 8, dans lequel le système de mesure comprend du 1,1-ferrocènediméthanol en tant que médiateur électronique.

10. Système de mesure selon l'une quelconque des revendications 5 à 9, dans lequel le système de mesure comprend un polymère rédox ayant un complexe d'osmium comme médiateur d'électrons.

11. Système de mesure selon l'une quelconque des revendications 5 à 10, dans lequel l'analyte (11) est du glucose, du lactose et/ou du phosphate, dans lequel l'enzyme de détection pour l'oxydation de l'analyte comprend de la glucose oxydase, de la lactose oxydase ou une combinaison de xanthine oxydase et de purine nucléoside phosphorylase.

12. Procédé pour la détermination électrochimique d'une concentration d'un analyte (11) dans une solution aqueuse avec un système de mesure selon l'une quelconque des revendications 5 à 11, **caractérisé en ce que** l'oxygène dissous (12) est éliminé dans la solution aqueuse avec un procédé selon l'une quelconque des revendications 1 à 4.

13. Procédé selon la revendication 12, dans lequel l'analyte (11) est oxydé par catalyse par l'oxydase dans une réaction d'oxydation, dans lequel des électrons (5) sont transportés de la réaction d'oxydation du médiateur d'électrons à une électrode (4), dans lequel le courant résultant du transport des électrons (5) est mesuré à travers le médiateur d'électrons.

14. Procédé selon la revendication 12 ou 13, dans lequel l'analyte (11)
- est du glucose, dans lequel l'oxydase utilisée est la glucose oxydase ; ou
- du lactose ou du phosphate, dans lequel l'oxydase utilisée est la lactate oxydase ou une combinaison de xanthine oxydase et de purine nucléoside phosphorylase.

15. Procédé selon l'une quelconque des revendications 12 à 14, dans lequel le médiateur électronique
- utilisé est du 1,1-ferrocènediméthanol ; ou
- un polymère rédox contenant un complexe d'osmium est utilisé.
